# EUROPEAN PATENT APPLICATION

(11) **EP 3 208 784 A1**
(43) Date of publication of application: **23.08.2017**
(21) Application number: 15851174.1
(22) Date of filing: 03.02.2015
(51) Int. Cl.: G08B 1/08

(54) **INTELLIGENT WEARABLE DEVICE AND SYSTEM HAVING ALARM FUNCTION**

(30) Priority: 15.10.2014 CN 201410543332
(71) Applicant: Qu, Honggui, Beijing 100095 (CN)
(72) Inventor: ZHOU, Chao, Beijing 100095 (CN)
(74) Representative: Biallo, Dario
(86) International application number: PCT/CN2015/072132
(87) International publication number: WO 2016/058298

(57) **Abstract**

An intelligent wearable device (1) and system having an alarm function, comprising: an alarm detection apparatus (11), a main control apparatus (12), an evidence collection apparatus (14) and an alarm transmission apparatus (13). When the alarm detection apparatus (11) determines that an abnormal situation has occurred, the alarm detection apparatus (11) sends an alarm signal to the main control apparatus (12); the main control apparatus (12) alarms according to the alarm signal; and the evidence collection apparatus (14) acquires evidence collection information, and the alarm transmission apparatus (13) sends the alarm information and evidence collection information to a background operation server (2). The intelligent wearable device (1) and system support various alarm ways, automatically send the alarm information to the background operation server (2) and can also send the alarm information to a designated contact person at the same time, the background operation server (2) sends the alarm information to a public security department, a medical department, a fire department, a traffic management department and so on so as to handle the warning condition, an environment safety monitoring function and a food safety monitoring function are achieved, and the situation that no person accepts the alarm can be avoided.

## Description

### Background of the Invention

### Technical Field of the Invention

The present invention relates to the technical field of a wearable device, and in particular an intelligent wearable device and system having an alarm function.

### Description of Related Art

Safety demand is a primary demand for personal survival. Ensuring people's personal safety is a significant factor of public order maintenance and social stability assurance. In case of an emergency or danger, the public generally ask for help by means of call alarm. In case of situations like kidnap, hijack, sudden disease, etc. that mobile communication devices cannot be used, or in case where call alarm operation is unavailable for children or old people, it is likely to cause a misery because no alarm can be issued in time. Due to the above causes, some portable devices having an alarm function are introduced into the market, and they are small in size, highly hidden and convenient for individuals to carry. For example, a wearable device, which is a portable device that is directly worn on body or integrated into a user's clothing or accessories, may realize powerful functions by software support and data interaction or cloud interaction. Currently, an existing portable device having an alarm function on the market generally has a single alarm function, less alarm trigger modes, less alarm information report modes, and delayed alarm processing, which leads to the actual applications cannot achieve the desired application effects. The existing portable devices having an alarm function have the following drawbacks.

Single Alarm Function: currently, portable devices having an alarm function on the market have a single alarm function and less alarm trigger modes, and fail to cope with various emergencies; a one-touch alarm function is widely used on the market, with which an alarm is issued by triggering a button; however, such alarm mode cannot be effectively triggered in time if the user is controlled.

Less Alarm Report Modes: currently, portable devices having an alarm function on the market generally have such alarm report mode as to report an alarm via a mobile communication network, but such report mode cannot be effectively achieved in time, in case of bad network signals or area without network coverage.

Delayed Alarm Processing: currently, portable devices having an alarm function on the market generally report alarm information to a designated contact person who is up to further process the alarm information (e.g. call 110 for help); when it is inconvenient to receive the alarm information or it is impossible to further process the alarm information for the specified contact person, no effective help can be given in time to the user.

### Summary of Invention

In view of the above, the present invention seeks to solve a technical problem of providing an intelligent wearable device having an alarm function, wherein alarm information is transmitted to a background operation server after alarm is triggered.

In an embodiment according to the present invention, an intelligent wearable device having an alarm function comprises a main control apparatus for performing an alarming processing in accordance with an alarm signal; an alarm detection apparatus for transmitting the alarm signal to the main control apparatus upon determining that an abnormal situation occurs; an evidence collection apparatus for acquiring evidence collection information, including audio, video, pictures, and characters, in accordance with a control command transmitted from the main control apparatus; and an alarm transmission apparatus for transmitting alarm information and the evidence collection information to a background operation server in accordance with the control command transmitted from the main control apparatus, wherein the main control apparatus is electrically connected to the alarm detection apparatus, the evidence collection apparatus, the alarm transmission apparatus, respectively.

In an embodiment according to the present invention, further, the alarm detection apparatus comprises an alarm button, electrically connected to the main control apparatus and transmitting an alarm signal to the main control apparatus when a user presses the alarm button.

In an embodiment according to the present invention, further, the alarm detection apparatus comprises a heart rate monitoring unit electrically connected to the main control apparatus, the heart rate monitoring unit comprising a pulse sensor, by which irradiation light is transmitted to a human's wrist, and reflected light obtained by being attenuated and absorbed by a human tissue is converted into an electrical signal; the heart rate monitoring unit amplifies and shapes the electrical signal to obtain a pulse signal in synchronization with a human pulse; if the heart rate monitoring unit determines that a frequency of the pulse signal exceeds a preset frequency safety threshold value, the heart rate monitoring unit transmits the alarm signal to the main control apparatus.

In an embodiment according to the present invention, further, the alarm detection apparatus comprises an audio processing unit electrically connected to the main control apparatus and used for transmitting the alarm signal to the main control apparatus, upon determining that a decibel value of environmental sound is higher than the preset high decibel threshold value or a set keyword occurs in the environmental sound; and a pickup electrically connected to the audio processing unit for collecting the environmental sound in real time and transmitting the environmental sound to the audio processing unit.

In an embodiment according to the present invention, further, the alarm detection apparatus comprises a positioning unit electrically connected to the main control apparatus and acquiring a user's position using the China Beidou navigation satellite system (BDS) or the GPS, and transmitting the position information to the main control apparatus, the main control apparatus performing the alarming processing when the main control apparatus determines that the user's position exceeds a set safe zone.

In an embodiment according to the present invention, further, the alarm detection apparatus comprises an oscillation detecting unit electrically connected to the main control apparatus and comprising an oscillation sensor for converting a collected mechanical quantity into an electrical signal, and amplifies the electrical signal before outputting a voltage signal; the oscillation detecting unit transmits a low level alarm signal to the main control apparatus upon determining that a voltage value of the voltage signal exceeds a present voltage safety threshold value.

In an embodiment according to the present invention, further, the alarm detection apparatus comprises a gas sensing unit electrically connected to the main control apparatus and comprising a gas sensor for converting concentration information of an odor or toxic and harmful gas in the environmental air into an electrical signal, in which the toxic and harmful gas includes formaldehyde, benzene, or alcohol gas, the odor includes a small resulting from food spoilage or pesticide residue; the gas sensing unit transmits an alarm signal to the main control apparatus controlling the intelligent wearable device to oscillate upon determining that a voltage value of the electrical signal exceeds a preset safety threshold value.

In an embodiment according to the present invention, further, the evidence collection apparatus comprises an audio processing unit electrically connected to the main control apparatus; and a pickup electrically connected to the audio processing unit for collecting environmental sound in real time and transmitting the environmental sound to the audio processing unit, after the main control apparatus receives the alarm information, the audio processing unit encoding and compressing the environmental sound as transmissible audio evidence collection information.

In an embodiment according to the present invention, further, the evidence collection apparatus comprises: a camera for collecting in real time video data in RAW format; and a video collecting unit electrically connected to the main control apparatus and transmitting in real time the video data to the main control apparatus, the main control apparatus encoding and compressing the video data as transmissible video or picture evidence collection information.

In an embodiment according to the present invention, further, the alarm transmission apparatus comprises a communicating unit electrically connected to the main control apparatus and comprising a 2G/3G wireless module, a GPRS module, or a wifi module capable of transmitting alarm information including characters, video, pictures, voice information, BDS positioning and short message information, and GPS positioning information to the background operation server in various communication modes.

In an embodiment according to the present invention, further, the main control apparatus controls the communication module to transmit the alarm information and the evidence collection information to a preset contact person when receiving the alarm signal.

In an embodiment according to the present invention, further, a storing unit electrically connected to the main control apparatus, providing a Micro-SD interface and capable of locally storing the alarm information and the evidence collection information; and a display unit electrically connected to the main control apparatus and comprising a touch screen for displaying information including characters images and video and transmitting user's setting information to the main control apparatus.

In an embodiment according to the present invention, an intelligent wearable device system comprises an intelligent wearable device having an alarm function according to any embodiment above; and a background operation server for issuing an alarm in accordance with alarm information.

In an embodiment according to the present invention, further, embodiment.

In an embodiment according to the present invention, further, the background operation server analyzes the alarm information, and transmits the alarm information to alarm processing systems including a public security department system, a medical department system, and a fire department system.

The intelligent wearable device and system having an alarm function according to the present invention supports various alarm modes, and can automatically transmit alarm information to the background intelligent alarm system operation center. The intelligent alarm system operation center analyzes and stores the alarm information, and transmits it to a public security department, a medical department, a fire department, and a traffic management and so on so as to process the warning condition. After the alarm is triggered, information is transmitted to a designated contact person via the mobile network at the same time. Both of an environment safety monitoring function and a food safety monitoring function can be achieved. The present invention remedies the drawbacks that currently the intelligent device lacks full safety alarm measures and that the alarm apparatus has a single alarm trigger mode on the market, and avoids the situation that no person accepts the alarm or the alarm cannot be issued because of an unsmooth signal. The present invention has full alarm functions, intelligent alarm modes, wide trigger modes, effective alarm modes, as well as hidden alarm mode and intellectualized products.

### Brief Description of Drawings

The drawings as explained herein are used to provide a further comprehension of the present invention, and serve as a part of the present application. Exemplary embodiments and explanations of the present application are used to explain the present invention and do not serve as an improper limitation for the present invention. In the drawings,
Fig. 1 is a schematic diagram of one embodiment of the intelligent wearable device having an alarm function according to the present invention;
Fig. 2 is a schematic diagram of another embodiment of the intelligent wearable device having an alarm function according to the present invention;
Fig. 3 is a schematic diagram of operation of a warning condition of the intelligent wearable device having an alarm function according to the present invention;
Figs. 4A to 4C are schematic diagrams of a further embodiment of the intelligent wearable device having an alarm function according to the present invention, wherein Fig. 4A is a back schematic diagram, Fig. 4B is a front schematic diagram, and Fig. 4C is an undersurface schematic diagram.

### Detailed Description of Preferred Embodiment

Hereinafter, a more comprehensive description is given to the present invention with reference to the drawings, including exemplary embodiments for explanations of the present invention. Combining the drawings accompanying the embodiments of the present invention, a clear and complete description is given to technical solutions in the embodiments of the present invention below. Obviously, the embodiments as described are only a portion of embodiments of the present invention, instead of full embodiments. Based on the embodiments of the present invention, all the other embodiments obtained by those skilled in the art without any inventive effort, fall into the scope where protection is sought by the present invention. By combining the drawings and embodiments, the technical solutions of the present invention are described below in aspects.

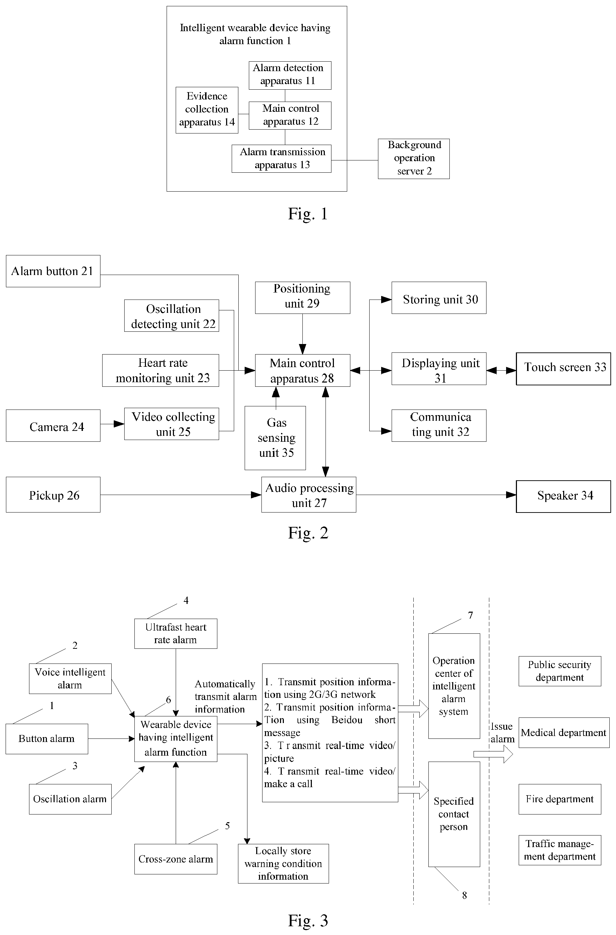

The present invention provides an intelligent wearable device having an alarm function. As shown in Fig. 1, intelligent wearable device 10 comprises: alarm detection apparatus 11, main control apparatus 12, evidence collection apparatus 14, and alarm transmission apparatus 13. Main control apparatus 12 is electrically connected to alarm detection 11, evidence collection apparatus 14 and alarm transmission apparatus 13, respectively.

If alarm detection apparatus 11 determines that an abnormal situation occurs, alarm detection apparatus 11 transmits an alarm signal to main control apparatus 12. Main control apparatus 12 performs an alarming processing in accordance with the alarm signal. Evidence collection apparatus 14 acquires audio, video or other evidence collection information in accordance with a control command transmitted from main control apparatus 12. Alarm transmission apparatus 13 transmits the alarm information and the evidence collection information in accordance with the control command transmitted from main control apparatus 12 to background operation server 20.

Intelligent wearable device 10 having an alarm function according to the present invention can support various alarm modes such as button alarm, voice intelligent alarm, oscillation alarm, heart rate alarm, or cross-zone alarm. Moreover, intelligent wearable device 10 can automatically transmit alarm information after triggering the alarm, for example transmit position information using the 2G/3G network or the BeiDou short message, transmit real-time video or pictures, transmit real-time audio information, make calls or transmit a character short message to the intelligent alarm system operation center (background operation server 20).

Background operation server 20 receives the alarm information and then analyzes, stores and transmits the alarm information to a public security department, a medical department, a fire department, and a traffic management and so on so as to process the warning condition, thus resulting in full alarm functions and intelligent alarm modes as well as hidden alarm modes and intellectualized products.

As shown in Fig. 2, the alarm detection apparatus comprises: alarm button 21, heart rate monitoring unit 23, audio processing unit 27, pickup 26, positioning unit 29, oscillation detecting unit 22, gas sensing unit 35, and so on.

Alarm button 21 is electrically connected to main control apparatus 28. When a user presses alarm button 21, alarm button 21 transmits an alarm signal to main control apparatus 28. Alarm button 21 may be used for one-touch trigger alarm, and alarm information is transmitted to main control apparatus 28 to be processed.

Heart rate monitoring unit 23 is electrically connected to main control apparatus 28. Heart rate monitoring unit 23 comprises a pulse sensor. The pulse sensor is used to transmit irradiation light to human's wrist, and convert reflected light, obtained by being attenuated and absorbed by a human tissue, into an electrical signal. Heart rate monitoring unit 23 amplifies and shapes the electrical signal to obtain a pulse signal in synchronization of a human pulse. If the heart rate monitoring unit 23 determines that a frequency of the pulse signal exceeds the preset frequency safety threshold value, e.g. 100, heart rate monitoring unit 23 transmits an alarm signal to main control apparatus 28. The pulse sensor is a core part positioned on a wrist strap of the apparatus. Green light or red light may be selected as the irradiation light source.

Pickup 26 is electrically connected to audio processing unit 27, and audio processing unit 27 is electrically connected to main control apparatus 28. Pickup 26 collects in real time environmental sound and transmits the environmental sound to audio processing unit 27. If audio processing unit 27 determines that a decibel value of the environmental sound is higher than the preset high decibel threshold value or the set keyword occurs in the environmental sound, audio processing unit 27 transmits an alarm signal to main control apparatus 28. Speaker 34 outputs audio information in accordance with a control command transmitted by the main control apparatus.

The chips of the audio processing unit 27 mainly includes an audio codec and a digital signal processor (DSP). For example, it is implemented by an audio chip having an Line In audio sampling interface (interfacing the pickup) and a Speak Out audio output interface (interfacing the speaker), which embeds the collected adaptive differential pulse code modulation (ADPCM) audio data with a particular algorithm using the DSP so as to recognize and analyze audio decibel and content, and transmit the analysis result to main control apparatus 28.

Video collecting unit 25 is electrically connected to main control apparatus 28. Video collecting unit 25 transmits in real time video data in RAW format, as collected by camera 24, to main control apparatus 28, and main control apparatus 28 encodes and compresses the video data. Video collecting unit 6 is used for collecting video information in real time using camera 2.

Positioning unit 29, which may be the China BeiDou Navigation Satellite System (BDS) module or the U.S. global positioning system (GPS) module, acquires position information and transmits it to main control apparatus 28.

Positioning unit 29 is electrically connected to main control apparatus 28. Positioning unit 29 acquires user's position information in real time using the China BeiDou Navigation Satellite System (BDS) or the GPS, and transmits the position information to main control apparatus 28. If main control apparatus 28 determined that the user's position information exceeds the set safe zones, main control apparatus 28 issues an alarm.

Oscillation detecting unit 22 is electrically connected to main control apparatus 28. Oscillation detecting unit 22 comprises an oscillation sensor. The oscillation sensor is used to convert a collected mechanical quantity into an electrical signal, and oscillation detecting unit 22 amplifies the electrical signal before outputting a voltage signal. If oscillation detecting unit 22 determines that a voltage value of the voltage signal exceeds the preset voltage safety threshold value, oscillation detecting unit 22 transmits a low level alarm signal to main control apparatus 28.

The oscillation sensor is a core part. A photoelectric oscillation sensor may be used to collect a mechanical quantity and convert the mechanical quantity into an electrical signal. The measurement line amplifies the electrical signal and outputs a voltage signal. If a value of the voltage signal exceeds the set safety value, a low level signal is outputted to main control apparatus 28 for alarm processing.

Gas sensing unit 35 is electrically connected to main control apparatus 28. Gas sensing unit 35 comprises a gas sensor. The gas sensor is used to convert concentration information of an odor or toxic and harmful gas in the collected environmental air into an electrical signal. If gas sensing unit 35 determines that a voltage value of the electrical signal exceeds the preset safety threshold value, gas sensing unit 35 transmits an alarm signal to main control apparatus 28, and main control apparatus 28 controls the intelligent wearable device to oscillate. The toxic and harmful gas includes: formaldehyde, benzene, alcohol or the like gas. The odor includes a smell resulted from food spoilage, pesticide residue or the like.

Gas sensing unit 35 has an environment safety monitoring alarm function and a food safety monitoring alarm function, and can detect the environment situation and the food safety situation according to user needs. If toxic and harmful gas exists in the environment or food spoilage and abnormality is detected, the device oscillates to remind the user to keep away as soon as possible.

The evidence collection apparatus comprises: camera 24, video collecting unit 25, and pickup 26. Pickup 26 is electrically connected to audio processing unit 27, and audio processing unit 27 is electrically connected to main control apparatus 28. After main control apparatus 28 receives the alarm information, pickup 26 collects environmental sound in real time and transmits the environmental sound to audio processing unit 27 to be encoded and compressed as transmissible audio evidence collection information.

The alarm detection apparatus and the evidence collection apparatus may share the pickup, the audio processing unit and other components, alternatively, the alarm detection apparatus and the evidence collection apparatus may be provided with pickup, audio processing unit and other components, separately.

Video collecting unit 25 is electrically connected to main control apparatus 28. Video colleting unit 25 transmits, in real time, video data in RAW format, as collected by camera 24, to main control apparatus 28 which, then, encodes and compresses the video data. Video collecting unit 6 is used for collecting video information in real time using camera 2.

A core chip of video collecting unit 25 may be a complementary metal-oxide semiconductor (CMOS) or charge coupled device (CCD) image sensor. Video collecting unit 25 can transmit the video data in RAW format, as collected in real time, to main control apparatus 28 to be encoded and compressed, using a mobile industry processor interface (MIPI).

The alarm transmission apparatus comprises communicating unit 32. Communicating unit 32 is electrically connected to main control apparatus 28. Communicating unit 32 comprises: a 2G/3G wireless module, a GPRS module, a wifi module, and other modules. Communicating unit 32 can transmit alarm information to the background operation server in various communication modes.

After the alarm is triggered, the apparatus automatically transmits alarm information, which comprises transmitting position information using the 2G/3G network, transmitting position information using the Beidou short message, transmitting real-time video and pictures, transmitting real-time audio information, making calls or transmitting a character short message to the intelligent alarm system operation center. When main control apparatus 28 receives the alarm signal, it controls communicating unit 32 to transmit the alarm information to a preset contact person.

In one embodiment, the intelligent wearable device having an alarm function has both a clock display function and a mobile communication function. The intelligent wearable device may acquire and transmit characters, video, pictures, or voice information using the 2G/3G network, support the BDS positioning and short message function as well as the GPS, have a touch display screen, and have a local storage function.

Main control unit 28 is electrically connected to storing unit 30 and displaying unit 31, respectively. Storing unit 30 provides a Micro-SD interface which supports the insertion of the SD card to extend local storage space and locally store the alarm information and the evidence collection information. Displaying unit 31 comprises touch screen 33. Touch screen 33 is used for displaying information, and transmitting the user's setting information to main control apparatus 28, the information including characters, images, video and so on.

In one embodiment, main control apparatus 28 is implemented by an encoding chip in ARM architecture. Main control apparatus 28 is based on the linux development platform and has a built-in Android operating system. It may compress and encode a video signal and an audio signal. Generally, a compression method of the video signal comprises hardware encoding compression and software compression, and its encoding standard comprises various formats such as H.264. A compression method of the audio signal comprises hardware encoding compression and software compression, and its encoding standard comprises various formats such as G.711. The encoded video/audio data, together with analysis data such as button, oscillation, heart rate and audio, may be transmitted by the communicating unit to the intelligent alarm operation center.

The intelligent wearable device according to the present invention may be in two states, respectively of a safe state and a trigger state. For instance, in a safe state, the touch liquid crystal display screen displays a clock interface, whereby to set a tab to enter the Android operating system interactive interface, so as to achieve an arbitrary call function, a limited call function, a wireless earphone call, a pedometer or other mobile communication function.

A positioning system is provided within the intelligent wearable device and comprises the GPS or the DBS which feeds back the user personal positioning information, personal activity map information, and personal setting information to the intelligent alarm system operation center as records. A built-in environment safety and food safety detection application achieves real-time monitoring and manual detection modes in accordance with user setup.

When an environment safety and food safety state detection is performed, a gas hole at the bottom of the device is opened to collect and analyze the environmental air quality and the taste of food nearby. If formaldehyde, benzene, alcohol, or other toxic and harmful gas exists in the environment or food emits an odor (spoilage or pesticide residue), the device oscillates to remind the user. The device has full alarm functions and intelligent alarm modes as well as intellectualized products.

Fig. 3 is a schematic diagram of operation of a warning condition of the intelligent wearable device having an alarm function according to the present invention. As shown in Fig. 3, if alarm button trigger, voice abnormity (include high decibel alarm and keyword alarm), oscillation abnormity, heart rate abnormity, or personal positioning information abnormity occurs, alarm is triggered to enter a warning condition. In accordance with the user setup of the warning condition, a camera is turned on to capture and transmit video and pictures information, and a pickup is turned on to transmit real-time audio information. Position information is transmitted to the intelligent alarm system operation center, and meanwhile such setup can be made such as to made calls or transmit information to a specified contact person. All the information and records are locally stored in synchronization.

Button alarm 1 achieves one-touch alarm by triggering a hidden alarm button by user. Voice intelligent alarm 2 includes high decibel voice alarm and keyword voice alarm. Environmental sound is detected and analyzed, if a sound, whose decibel is higher than the set decibel value, occurs, or if the set keyword occurs, alarm is issued. Oscillation alarm 3 may issue an alarm by detecting an abnormal oscillation undergone by the apparatus. Ultrafast heart rate alarm 4 may monitor user's heart rate value in real time by the apparatus, and if a heart rate abnormity occurs, an alarm is issued. Cross-zone alarm 5 may record user position information and activity map in real time by the GPS or BDS, and if the set safe zone is exceeded, an alarm is issued.

When the intelligent wearable device is used to issue an alarm, the user's position information, video, pictures, characters or voice information is transmitted to a specified contact person and to the intelligent alarm system operation center 7 using the 2G/3G network; the intelligent alarm system operation center 7 supports both transmission and reception of the Beidou short message; finally, alarm information is submitted with the public security, fire, hospital, traffic management and the like department to complete the alarm. The alarm information may be locally stored.

The intelligent wearable device of the present invention can avoid the situation that no person accepts the alarm or that alarm cannot be issued because of unsmooth signal. Compared with the existing technologies on the market, the present invention has full alarm functions, intelligent alarm modes, wide trigger modes, effective alarm modes, as well as hidden alarm modes and intellectualized products.

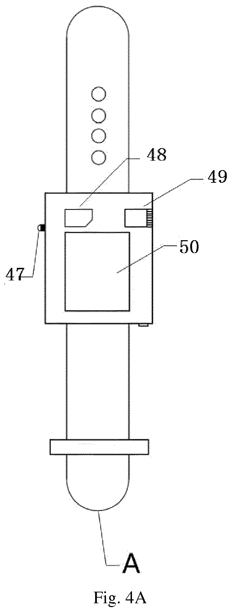

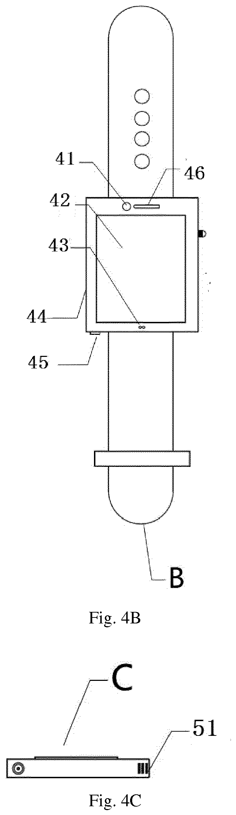

As shown in Figs. 4A-4C, the intelligent wearable device having an alarm function as concerned in the present invention has a wearable shape like watch, necklace, or pendant, is portable and hidden, and waterproof and dustproof. For example, the watch shape has a time display function as well as a communication function, a network function and etc. of the intelligent watch, has five alarm function trigger modes including button alarm, intelligent voice alarm, oscillation alarm, heart rate alarm and cross-zone alarm, as well as both the environment safety monitoring and food safety monitoring functions.

The intelligent wearable device in a watch shape has camera 1, touch screen 2, microphone 3, shell 4, earphone jack 5, speaker 6, alarm button 7, SIM card slot 8, SD card slot 9, battery installation slot 10, and gas sense hole 11. Camera 1 is used for collecting image information; touch screen 2 is used for display setup of an graphical interface, character recording, video image playing, and etc.; microphone 3 is used for collecting audio information; shell 4 is a waterproof dustproof shell; earphone jack 5 supports 3.5 mm earphone communication; speaker 6 outputs audio; alarm button 7 is a hidden alarm button and achieves a one-touch alarm function; SIM card slot 8 is compatible with installation of the SIM or Micro-SIM card; the SD card slot 9 is used for installing the Micro-SD card so as to extend storage capacity; battery installation slot 10 is used for installation and replacement of a lithium battery so as to supply power to the apparatus; gas sense hole 11 is designed as shutters to open or close the gas hole in accordance with user needs, and is used for achieving the gas sensing function.

In one embodiment, the present invention provides an intelligent wearable device system, comprising: a background operation server, i.e. the intelligent alarm system operation center, and an intelligent wearable device having an alarm function as above. The background operation server process the alarm in accordance with alarm information. The background operation server stores and analyzes the received alarm information, and transmits the alarm information to various alarm processing systems in accordance with the analysis result. The alarm processing systems include a public security department system, a medical department system, a fire department system, a traffic management department system, and so on.

The intelligent wearable device and system having an alarm function, as provided in the aforementioned embodiment, supports various alarm modes. After alarm is triggered, the device automatically transmits alarm information to the intelligent alarm system operation center. Upon receipt of the alarm information, the intelligent alarm system operation center analyzes and stores the alarm information, and transmits it to a public security department, a medical department, a fire department, and a traffic management department, and so on to process the warning condition. A specific contact person may be set, and meanwhile the information is transmitted to the specified contact person via the mobile network after the alarm is triggered. A local storage function is supported. Both of an environment safety monitoring function and a food safety monitoring function are achieved. In case of a bad environmental air quality or a food odor, the device oscillates to remind the user.

The intelligent wearable device and system having an alarm function, as provided in the aforementioned embodiment, remedies the drawbacks of the lack of full safety protection alarm measures of the intelligent device on the market and a single trigger alarm mode of the alarm apparatus. Meanwhile the intelligent alarm system operation center is established. Alarm information is transmitted not only to a specified contact person but directly to a private operation center by means of the 2G/3G network or the Beidou short message. It is the operation center that directly manages user information and process the alarm information, thus avoiding the situation that no person accepts the alarm or alarm cannot be issued because of unsmooth signal. Compared with the existing technologies on the market, the device and system have full alarm functions, intelligent alarm modes, wide trigger modes, effective alarm modes, as well as hidden alarm modes and intellectualized products.

It is likely to implement the method and system of the present invention in many ways, for example, by any or a combination of software, hardware and firmware. The aforementioned order of method steps is only for explanations, and the method steps of the present invention are not limited to the above-described order, unless otherwise specified. Besides, in some embodiments, it is further likely to implement the present invention as programs recorded in the recording medium, and these programs include machine-readable instructions for implementing the method according to the present invention. Therefore, the present invention further covers a recording medium that stores a program for executing the method according to the present invention.

Description of the present invention is given for the purpose of illustration and description, and it is not exhausted or makes the present invention limited to the disclosed patterns. Modifications and changes are obvious for those skilled in the art. Selections and descriptions of embodiments are for the purpose of better explaining principles and actual applications of the present invention, and enabling those skilled in the art to understand the present invention so as to design various embodiments with various modifications adaptive for particular usage.

## Claims

1. An intelligent wearable device having an alarm function, comprising:
a main control apparatus for performing an alarming processing in accordance with an alarm signal;
an alarm detection apparatus for transmitting the alarm signal to the main control apparatus upon determining that an abnormal situation occurs;
an evidence collection apparatus for acquiring evidence collection information, including audio, video, pictures, and characters, in accordance with a control command transmitted from the main control apparatus; and
an alarm transmission apparatus for transmitting alarm information and the evidence collection information to a background operation server in accordance with the control command transmitted from the main control apparatus,
wherein the main control apparatus is electrically connected to the alarm detection apparatus, the evidence collection apparatus, the alarm transmission apparatus, respectively.

2. The apparatus according to claim 1, wherein,
the alarm detection apparatus comprises an alarm button, electrically connected to the main control apparatus and transmitting an alarm signal to the main control apparatus when a user presses the alarm button.

3. The apparatus according to claim 1, wherein,
the alarm detection apparatus comprises a heart rate monitoring unit electrically connected to the main control apparatus, the heart rate monitoring unit comprising a pulse sensor, by which irradiation light is transmitted to a human's wrist, and reflected light obtained by being attenuated and absorbed by a human tissue is converted into an electrical signal;
the heart rate monitoring unit amplifies and shapes the electrical signal to obtain a pulse signal in synchronization with a human pulse;
if the heart rate monitoring unit determines that a frequency of the pulse signal exceeds a preset frequency safety threshold value, the heart rate monitoring unit transmits the alarm signal to the main control apparatus.

4. The apparatus according to claim 1, wherein,
the alarm detection apparatus comprises:
an audio processing unit electrically connected to the main control apparatus and used for transmitting the alarm signal to the main control apparatus, upon determining that a decibel value of environmental sound is higher than the preset high decibel threshold value or a set keyword occurs in the environmental sound; and
a pickup electrically connected to the audio processing unit for collecting the environmental sound in real time and transmitting the environmental sound to the audio processing unit.

5. The apparatus according to claim 1, wherein,
the alarm detection apparatus comprises a positioning unit electrically connected to the main control apparatus and acquiring a user's position using the China Beidou navigation satellite system (BDS) or the GPS, and transmitting the position information to the main control apparatus, the main control apparatus performing the alarming processing when the main control apparatus determines that the user's position exceeds a set safe zone.

6. The apparatus according to claim 1, wherein,
the alarm detection apparatus comprises an oscillation detecting unit electrically connected to the main control apparatus and comprising an oscillation sensor for converting a collected mechanical quantity into an electrical signal, and amplifies the electrical signal before outputting a voltage signal,
the oscillation detecting unit transmits a low level alarm signal to the main control apparatus upon determining that a voltage value of the voltage signal exceeds a present voltage safety threshold value.

7. The apparatus according to claim 1, wherein ,
the alarm detection apparatus comprises a gas sensing unit electrically connected to the main control apparatus and comprising a gas sensor for converting concentration information of an odor or toxic and harmful gas in the environmental air into an electrical signal, in which the toxic and harmful gas includes formaldehyde, benzene, or alcohol gas, the odor includes a small resulting from food spoilage or pesticide residue;
the gas sensing unit transmits an alarm signal to the main control apparatus controlling the intelligent wearable device to oscillate upon determining that a voltage value of the electrical signal exceeds a preset safety threshold value.

8. The apparatus according to claim 1, wherein,
the evidence collection apparatus comprises:
an audio processing unit electrically connected to the main control apparatus; and
a pickup electrically connected to the audio processing unit for collecting environmental sound in real time and transmitting the environmental sound to the audio processing unit, after the main control apparatus receives the alarm information,
the audio processing unit encoding and compressing the environmental sound as transmissible audio evidence collection information.

9. The apparatus according to claim 1, wherein,
the evidence collection apparatus comprises:
a camera for collecting in real time video data in RAW format; and
a video collecting unit electrically connected to the main control apparatus and transmitting in real time the video data to the main control apparatus, the main control apparatus encoding and compressing the video data as transmissible video or picture evidence collection information.

10. The apparatus according to claim 1, wherein,
the alarm transmission apparatus comprises a communicating unit electrically connected to the main control apparatus and comprising a 2G/3G wireless module, a GPRS module, or a wifi module capable of transmitting alarm information including characters, video, pictures, voice information, BDS positioning and short message information, and GPS positioning information to the background operation server in various communication modes.

11. The apparatus according to claim 10, wherein,
the main control apparatus controls the communication module to transmit the alarm information and the evidence collection information to a preset contact person when receiving the alarm signal.

12. The apparatus according to claim 1, further comprising:
a storing unit electrically connected to the main control apparatus, providing a Micro-SD interface and capable of locally storing the alarm information and the evidence collection information; and
a display unit electrically connected to the main control apparatus and comprising a touch screen for displaying information including characters images and video and transmitting user's setting information to the main control apparatus.

13. An intelligent wearable device system comprising:
an intelligent wearable device having an alarm function according to any one of claims 1 to 12; and
a background operation server for issuing an alarm in accordance with alarm information.

14. The system according to claim 13, wherein,
the background operation server stores the received alarm information.

15. The system according to claim 13 or 14, wherein ,
the background operation server analyzes the alarm information, and transmits the alarm information to alarm processing systems including a public security department system, a medical department system, and a fire department system.
